# EUROPEAN PATENT APPLICATION

(11) **EP 1 123 695 A2**
(43) Date of publication of application: **16.08.2001**
(21) Application number: 01200154.1
(22) Date of filing: 17.01.2001
(51) Int. Cl.: A61K 7/16

(54) **Oral composition comprising capsules containing keratin**

(30) Priority: 28.01.2000 EP 00200280
(71) Applicant: UNILEVER N.V., 3013 AL Rotterdam (NL); UNILEVER PLC, London EC4P 4BQ (GB)
(72) Inventor: Di-Drusco, Isotta, 20121 Milano (IT)
(74) Representative: Rosen Jacobson, Frans Lucas M.

(57) **Abstract**

Oral composition comprising capsules containing substantially water-insoluble keratin, characterised in that the capsules comprise from 0.05 to 1.5% by weight keratin.

## Description

The present invention relates to an oral composition which comprises capsules containing water-insoluble keratin.

Compositions for use in personal care, which comprise capsules containing keratin, are known in the art. JP 10/337,466 (Akusenu) discloses their use in cosmetic formulations. The capsules described therein comprise water soluble keratin as an improved wall material. The keratin is in the form of microfibrils of molecular weight between 35,000 and 60,000.

Further, our co-pending European patent application (99305224.0) also relates to an oral composition comprising keratin, wherein the keratin is in a substantially water-insoluble form.

While keratin is a desirable ingredient in personal product compositions it is preferred that it is the core of the capsule which contains keratin as it is the core which is released when the capsule is burst.

We have surprisingly found that the amount of keratin to be included in the encapsulation process can be modified to improve the breakage characteristics of the capsules. This is so even though the capsules have to be resilient enough to survive manufacturing processing.

Accordingly, a first aspect of the present invention provides an oral composition according to claim 1.

Capsules according to the invention typically comprise a core surrounded by an encapsulating wall. By containing keratin is meant that the keratin is contained in the core of the capsule so it can be released when the capsule wall is ruptured.

Such an insoluble form of keratin is available commercially from Freeman Chemical Corp.

In a particularly preferred embodiment the keratin is in the form of water insoluble particles the majority of which have a diameter no greater than 75 µm, more preferably, no greater than 50 µm. To obtain such small keratin particles it may be necessary to sieve them through a suitably gauged sieve.

The capsules according to the invention typically comprise from 0.05 to 1.5% by weight, preferably from 0.1 to 1.2% and especially preferably from 0.12 to 1.0% by weight of water-insoluble keratin.

In a particular embodiment the invention provides an oral composition comprising capsules which contain substantially water-insoluble keratin, characterised in that the capsules' average breaking force is between 0.06 to 0.2 N, preferably between 0.08 and 1.6 N, and especially preferably between 0.09 and 1.2 N.

In a particular embodiment the capsules according to the invention additionally comprise as core material an oil. Suitable oils include vegetable oils, mineral oils, silicone oils and hydrocarbon oils. Vegetable oils are particularly preferred, especially sunflower oil, safflower oil, rape seed oil and soybean oil.

Preferably the capsules' weight average particle size ranges from 600 to 1400 µm, more preferably from 700 to 1200 µm.

Typical encapsulating materials are common in the art and include but are not limited to cyclodextrin, gum arabic, gelatin, casein, albumin, fibrinogen, xanthan gum, haemoglobin, soluble collagen peptides, sodium alginate, carboxy-methyl cellulose, carrageenan, polyvinylpyrrolidone and similar natural or synthetic polymeric materials. Many suitable encapsulating materials are cross-linked. Cross-linking may be inherent in the encapsulating material or it may be achieved by a cross-linking agent, e.g. glutaraldehyde.

Typically, capsules according to the invention comprise, on average, from 1.5 to 8%, preferably 2 to 6% by weight of the encapsulating material. It is to be understood that the actual amounts present in the capsules may vary around this average.

A preferred capsule comprises an encapsulating material comprising both gelatin and gum arabic in a 0.8:1 to 1.2:1 ratio by weight and cross-linked with glutaraldehyde.

The capsules can be made by any conventional microencapsulation process, preferably by complex coacervation. The process conditions and encapsulating materials should be carefully chosen, so that capsules are obtained which are neither too hard to survive any crushing during use, nor too soft to crush already during manufacture of the oral care composition.

The encapsulating materials should be stable in the presence of anionic surfactants, e.g. sodium lauryl sulphate, which is commonly included in oral compositions as a foaming agent. Such encapsulating materials preferably include gum arabic, gelatin and mixtures and derivatives thereof.

Typical agents which may be encapsulated in addition to the keratin include any agent capable of exhibiting a therapeutic, sensory, protective or cosmetic effect and include antimicrobial agents, anti-caries agents, gum protection agents, flavours, colours, whitening agents and suchlike.

The particle size of the capsules can be measured using conventional methods, for example, standard gauge sieves and microscopy.

Typically, the encapsulated agent will comprise from 0.01 to 10%, preferably from 0.1 to 5% and more preferably from 0.1 to 2% by weight of the oral composition according to the invention.

The oral composition according to the invention comprise further ingredients which are common in the art, such as:
antimicrobial agents, e.g. Triclosan, chlorhexidine, copper, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
other proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.
humectants such as glycerol, sorbitol, propyleneglycol, xylitol, lactitol etc.;
binders and thickeners such as sodium carboxymethylcellulose, xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included. Examples of such polymers are copolymers of polyvinylmethylether with maleic anhydride and other similar delivery enhancing polymers, e.g. those described in DE-A-3,942,643 (Colgate);
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

The oral composition may be in any form common in the art, e.g. toothpaste, gel, mousse, aerosol, gum, lozenge, powder, cream, etc. and may also be formulated into systems for use in dual-compartment type dispensers.

In a preferred embodiment the oral composition according to the invention is a gel, preferably a visually clear gel.

The present invention will be further illustrated by way of example. The capsules are, preferably, made by complex coacervation with the sieved, water-insoluble keratin particles included in the coacervation reaction mixture.

### EXAMPLE 1

The following dentifrice formulation represents a formulation according to the invention and is manufactured by processes common in the art:

| | Mass % |
|---|---|
| Sorbitol syrup (70%) | 62.00 |
| Abrasive silica | 8.00 |
| Thickening silica | 8.00 |
| Polyethylene glycol (MW 1,500) | 4.00 |
| Sodium laurylsulphate | 1.80 |
| Flavour oil | 1.20 |
| Sodium monofluoro phosphate | 1.12 |
| Sodium saccharin | 0.20 |
| Capsules comprising from 0.05 to 1.5% by weight keratin (average weight particle size 800-1000 µm) | 1.00 |
| Thickener (SCMC) | 0.60 |
| Water | 12.08 |

### EXAMPLE 2

The capsules bursting force is measured by the following method.

A single capsule is placed on a base plate. A little punch which is mounted on a dynanometer having a low capacity cell (10 N) can be used to determine the force required to burst a capsule.

The apparatus is set up so as to crush the capsule at a cross-head velocity of 10 µm per second measuring the evolution of the force applied. Typically 20 capsules are burst and the results averaged to produce a bursting force.

The formulation of example 1 was prepared with two capsule types: type 'A' with capsules comprising 0.01% by weight of the keratin according to the invention and type 'B' comprising 0.2% by weight keratin.

The bursting force for A was 0.2 N and 0.1 N for B.

### EXAMPLE 3

The capsules' breakage during brushing was measured by counting the number of capsules remaining and was 45% and 64% for A and B respectively.

Thus, by increasing the level of keratin the breakage characteristics can be optimised thereby providing maximum release of the keratin into the oral cavity not only because more is contained in the capsules but also because more capsules are being broken.

## Claims

**1.** Oral composition comprising capsules, which capsules contain water-insoluble keratin, characterised in that the capsules comprise from 0.05 to 1.5% by weight of the keratin.

**2.** Composition according to claim 1, characterised in that the keratin is in the form of water insoluble particles, the majority of which are of diameter no greater than 75 µm.

**3.** Composition according to claim 2, characterised in that the keratin is in the form of water insoluble particles, the majority of which are of diameter no greater than 50 µm.

**4.** Composition according to any of claims 1 to 3, characterised in that the capsules further comprise as core material an oil.

**5.** Composition according to any preceding claim, characterised in that the capsules' average breaking force is from 0.06 to 0.2 N.

**5.** Process for the manufacture of capsules according to any preceding claim by complex coacervation.
